# EUROPEAN PATENT APPLICATION

(11) **EP 2 829 276 A1**
(43) Date of publication of application: **28.01.2015**
(21) Application number: 13764862.2
(22) Date of filing: 16.03.2013
(51) Int. Cl.: A61K 36/28, A61K 36/48, A61K 36/53, A61K 36/54, A61K 36/76, A61K 36/886, A61K 36/185, A61P 17/00, A61P 17/06

(54) **COMPOSITION FOR PREVENTING AND/OR TREATING DERMATOSIS AND METHOD FOR OBTAINING SAME**

(30) Priority: 22.03.2012 ES 201230434
(71) Applicant: Garcia Gilabert, Juan Miguel, 30860 Puerto De Mazarron (Mazarron) (ES)
(72) Inventor: Garcia Gilabert, Juan Miguel, 30860 Puerto De Mazarron (Mazarron) (ES)
(74) Representative: Hart, Deborah Mary
(86) International application number: PCT/ES2013/070178
(87) International publication number: WO 2013/140009

(57) **Abstract**

The invention relates to a composition for preventing and/or treating dermatosis, in particular psoriasis, and to a method for obtaining same, which includes chamomile, thyme, bay leaf, hops, millet, barley malt, brewer's yeast, willow, fenugreek *(Trigonella foenum-graecum),* salicaria *(Lythrum salicaria),* hibiscus *(Hibiscus),* olive oil, bee's honey, aloe vera, oat bran, chicory, coparchy *(Coutarea latifolia),* Stevia, wheat flour, cypress, brewer's malt, lanolin, elderberry, goldenrod *(Solidago),* witch-hazel, heal-all *(Prunella vulgaris),* and St John's wort *(Hypericum perforatum).*

## Description

### Purpose of the invention

The present invention relates to a composition for treating and preventing dermatosis, whether recent or old, and in particular psoriasis.

### Background of the invention

Abnormal scaly and/or keratotic formations of the dermis are manifestations of the condition of the skin that are encountered in many dermatoses, whether they are of recent appearance or are very old, in particular the various forms of psoriasis.

Psoriasis is a chronic progressive erythematosquamous dermatosis that comprises a plurality of red plaques covered with thick white scales and affects 2% of the population. With more than 60 000 new persons affected each year, it is one of the commonest dermatological disorders. This disorder is manifested by painful stinging and lesions that may cover more than 10% of the body. In its usual forms, its localization is very characteristic: elbows and cubital side of the forearms, knees, lumbosacral region, scalp and nails.

Some particular forms, described as severe psoriasis, are also known, such as erythrodermic psoriasis, psoriasis arthropathica or pustular psoriasis. Approximately 1.5% of psoriasis cases begin in childhood before the age of 10 years and 35% before the age of 20 years. This abnormality of renewal of the epidermis is therefore a serious disorder that represents an important public health problem.

The cause of psoriasis is unknown at present, but it is believed that multiple factors are involved. It has been possible to demonstrate a genetic factor, with 30% of psoriasis cases being familial, as well as some environmental factors. Psychological factors may often initiate some attacks. Skin injuries, alcohol and tobacco are some factors in severity and therapeutic resistance.

The evolution of the disease is chronic and occurs as attacks that are generally unforeseeable, interrupted by remissions during which the lesions are minimal. Even apart from the severe forms, and even though the patient's general condition is unchanged in the usual forms, psoriasis is a disease that may have profound effects on quality of life when the lesions are visible or uncomfortable for manual work, consequences that are often underestimated by the physician.

At present, physicians have at their disposal symptomatic treatments with temporary results, but not yet any effective medicinal products.

### Description of the invention

The aim of the present invention is to propose a composition for treating states caused by or that are manifested as abnormal keratotic and/or scaly formations of the dermis, such as those that are encountered in recent or chronic dermatosis and, in particular, in the various forms of psoriasis.

In a first aspect, the present invention relates to a composition for treating dermatosis (hereinafter composition of the present invention), characterized in that it comprises chamomile, thyme, laurel, hops, millet, barley malt, brewer's yeast, willow, fenugreek *(Trigonella foenum-graecum),* purple loosestrife *(Lythrum salicaria);* hibiscus *(Hibiscus),* olive oil, honey, aloe vera, oat bran, chicory, copalchi (Coutarea *latifolia), Stevia,* wheat flour, cypress, brewing malt, lanolin, elder, goldenrod *(Solidago), Hamamelis;* comfrey *(Prunella vulgaris),* and St. John's wort *(Hypericum perforatum).*

In a second aspect, the present invention relates to a method for obtaining the composition of the present invention, characterized in that it comprises grinding all the elements, except the aloe vera; then all the elements previously ground are diluted in water, leaving it to boil and to liquefy after it has boiled; and in that the aloe vera, on the other hand, is liquefied directly and is then mixed with the products previously boiled and liquefied, and the new mixture is liquefied again, leaving it to ferment in an open container prior to packaging.

Finally, it should be pointed out that the composition is especially useful for the topical treatment of psoriasis, but is also applicable for: spots on the skin, pimples, cellular regeneration, skin sweating and odors, skin tightening agent, chafing from footwear or some other type of chafing, antiwrinkle agents, acne, wound healing, eczemas, blackheads, hemorrhoids, burns, skin ulcers, chilblains, hematomas, insect bites, skin care, wounds and shaver's rash.

### Preferred embodiment of the invention and examples of use

In a preferred embodiment of the invention, the composition for preventing and/or treating dermatosis comprises diluting the following products in 40 liters of water: 150 g of chamomile; 150 g of thyme; 150 g of laurel; 150 g of hops; 150 g of millet; 200 g of barley malt; 150 g of brewer's yeast; 150 g of willow; 150 g of fenugreek *(Trigonella foenum-graecum);* 150 g of purple loosestrife *(Lythrum salicaria);* 200 g of hibiscus *(Hibiscus);* 1.5 liters of olive oil; 1 kg of honey; 5 kilos of aloe vera; 300 g of oat bran; 150 g of chicory; 150 g of copalchi *(Coutarea latifolia);* 200 g of *Stevia;* 1 kg of wheat flour; 150 g of cypress; 200 g of brewing malt; 150 cm³ of lanolin; 150 g of elder; 150 g of goldenrod *(Solidago);* 150 g of *Hamamelis;* 150 g of comfrey *(Prunella vulgaris);* and 150 g of St. John's wort *(Hypericum perforatum).*

The method of preparation is as follows:
In a first step, all the elements are ground, except the aloe vera. Then all the elements previously ground are diluted in boiling water (40 liters), for a period of time of between 10 and 12 minutes. Once the products have been boiled, they liquefy.

The aloe vera, on the other hand, is liquefied directly and is then mixed with the products previously boiled and liquefied, and the new mixture is liquefied again. The mixture is fermented for 3-4 days in an open container, prior to packaging.

The application or use of the product starts from topical application thereof on the affected zone, daily and for a period of between 2 and 3 months, noting improvement of the affected zone from ten or twelve days after application until complete disappearance of the scabs and scales typical of psoriasis toward the end of said period of application, observing complete disappearance of said scabs and scales and persistence of said disappearance, no reappearance of scabs and/or scales occurring in trials during the year following the end of treatment.

## Claims

1. A composition for preventing and/or treating dermatosis that comprises chamomile, thyme, laurel, hops, millet, barley malt, brewer's yeast, willow, fenugreek *(Trigonella foenum-graecum),* purple loosestrife *(Lythrum salicaria);* hibiscus *(Hibiscus),* olive oil, honey, aloe vera, oat bran, chicory, copalchi *(Coutarea latifolia), Stevia,* wheat flour, cypress, brewing malt, lanolin, elder, goldenrod *(Solidago), Hamamelis;* comfrey *(Prunella vulgaris),* and St. John's wort *(Hypericum perforatum).*

2. The composition as claimed in claim 1 for preventing and/or treating psoriasis.

3. A method for obtaining a composition as claimed in claim 1 for preventing and/or treating dermatosis that is **characterized in that** it comprises grinding all the elements, except the aloe vera; then all the elements previously ground are diluted in water, leaving it to boil and to liquefy after it has boiled; and **in that** the aloe vera, on the other hand, is liquefied directly and is then mixed with the products previously boiled and liquefied, and the new mixture is liquefied again, leaving it to ferment in an open container prior to packaging.

4. The use of the composition as claimed in claims 1 and 2 for making a medicinal product for treating psoriasis by topical application.

5. The use of the composition as claimed in claims 1 and 2 for preventing psoriasis by topical application.
